(19) Europäisches Patentamt
European Patent Office
Office européen des brevets

(11) **EP 2 678 734 B1**

(12) **FASCICULE DE BREVET EUROPEEN**

(45) Date de publication et mention de la délivrance du brevet:
**24.06.2020 Bulletin 2020/26**

(51) Int Cl.:
***G02C 13/00*** (2006.01)   ***A61B 3/11*** (2006.01)

(21) Numéro de dépôt: **11815765.0**

(22) Date de dépôt: **30.12.2011**

(86) Numéro de dépôt international:
**PCT/FR2011/000687**

(87) Numéro de publication internationale:
**WO 2012/113994 (30.08.2012 Gazette 2012/35)**

(54) **PROCÉDÉ DE DÉTERMINATION, EN POSTURE NATURELLE, D'AU MOINS UN PARAMÈTRE GÉOMÉTRICO-PHYSIONOMIQUE ASSOCIÉ AU MONTAGE D'UNE LENTILLE OPHTHALMIQUE DANS UNE MONTURE DE LUNETTES**

VERFAHREN ZUR BESTIMMUNG, IN EINER NATÜRLICHEN HALTUNG, VON MINDESTENS EINEM GEOMETRISCHEN/PHYSIOGNOMEN PARAMETER IM ZUSAMMENHANG MIT DER MONTAGE EINES BRILLENGLASES IN EINER BRILLENFASSUNG

METHOD FOR DETERMINING, IN A NATURAL POSTURE, AT LEAST ONE GEOMETRIC/PHYSIOGNOMIC PARAMETER ASSOCIATED WITH THE MOUNTING OF AN OPHTHALMIC LENS IN A SPECTACLE FRAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.02.2011 FR 1100511**

(43) Date de publication de la demande:
**01.01.2014 Bulletin 2014/01**

(73) Titulaire: **Essilor International 94220 Charenton-le-Pont (FR)**

(72) Inventeurs:
• **HADDADI, Ahmed**
  **F-94220 Charenton-le-Pont (FR)**

• **DELZERS, Jean**
  **F-94220 Charenton-le-Pont (FR)**

(74) Mandataire: **Jacobacci Coralis Harle 32, rue de l'Arcade 75008 Paris (FR)**

(56) Documents cités:
**WO-A1-2006/029875    WO-A1-2010/119190
WO-A1-2010/145736    WO-A2-2009/024681
FR-A1- 2 914 173    FR-A1- 2 915 290
JP-A- 2007 289 683    US-A1- 2007 195 266**

**Description**

DOMAINE TECHNIQUE AUQUEL SE RAPPORTE L'INVENTION

La présente invention concerne de manière générale un procédé de détermination d'au moins un paramètre géométrico-physionomique associé au montage d'une lentille ophtalmique dans une monture de lunettes portée par un porteur.

ARRIERE-PLAN TECHNOLOGIQUE

La confection d'une lentille correctrice pour lunettes comporte, d'une part, la conception optique et la mise en forme des faces de réfraction de la lentille et, d'autre part, l'adaptation de la lentille à la monture de lunettes choisie.

La présente invention traite de la mesure, sur le visage du porteur, de paramètres géométrico-physionomiques rendant compte de la configuration d'implantation des lunettes sur le visage du porteur. Ces paramètres sont susceptibles d'être exploités dans les deux étapes de confection d'une lentille correctrice, afin que la lentille exerce finalement la fonction optique corrective pour laquelle elle a été conçue et prescrite. Il s'agit notamment de la distance interpupillaire, de la hauteur des pupilles des yeux du porteur par rapport au bord inférieur de la monture, et/ou de l'angle pantoscopique que forme le plan général de la monture ou de la lentille par rapport à la verticale.

Les paramètres géométrico-physionomiques recherchés sont liés à la fois à la géométrie de la tête du porteur et de la monture de lunettes choisie, ainsi qu'à la posture du porteur.

De manière connue, il est possible de déterminer ces paramètres manuellement. Par exemple, la mesure de la hauteur des yeux du porteur par rapport au bord inférieur de la monture peut être effectuée par l'opticien qui observe le porteur de face et réalise une mesure à l'estime, au moyen d'un réglet, de la distance entre la pupille d'un oeil et le bord inférieur de la lentille de présentation.

Il est également possible de déterminer ces paramètres à partir d'une ou plusieurs images capturées de la tête du porteur.

Cependant, qu'elles soient manuelles ou automatisées, ces méthodes de détermination des paramètres géométrico-physionomiques du porteur souffrent d'une grande imprécision. En effet, pour effectuer une mesure précise au réglet ou par traitement des images capturées, il faut que la tête du porteur soit dans sa posture naturelle au moment de la mesure ou de la capture d'images.

Cette posture naturelle est aussi appelée posture anatomique ou position orthostatique. Dans cette posture naturelle, qui sera définie plus en détail ultérieurement, le porteur tient sa tête droite et regarde au loin, à l'horizon.

Pour des raisons de rapidité d'exécution et de confort de l'opticien comme du porteur, la position de la tête du porteur lors de la prise d'image n'est pas imposée.

Cependant, même si la position de la tête du porteur n'est pas imposée, celui-ci ne se trouve généralement pas dans la posture naturelle pendant la détermination des paramètres géométrico-physionomiques.

En effet, la mise en œuvre de ces méthodes de détermination des paramètres géométrico-physionomiques implique d'approcher un appareil de mesure à une distance faible, généralement inférieure à un mètre, du porteur. L'opticien s'approche également dans le cas d'une mesure manuelle.

Le porteur regarde alors cet appareil de mesure ou l'opticien qui se trouve alors devant lui pendant la mesure.

La proximité de l'appareil ou de l'opticien fait qu'en pratique, le porteur se cambre légèrement en arrière.

Le porteur se trouve alors dans une posture contrainte et non naturelle. Son regard n'est pas dirigé droit devant lui, à l'horizon, mais fixé sur un objet proche.

Or, une déviation de 1 degré de l'inclinaison de la tête dans son plan sagittal par rapport à la posture anatomique introduit une erreur de 1 degré sur la mesure de l'angle pantoscopique et une erreur de 0,5 millimètre sur la mesure des hauteurs des yeux.

Par ailleurs, la détermination desdits paramètres géométrico-physionomiques à partir d'images capturées implique l'identification, sur l'image capturée, de l'image d'au moins un élément de repère disposé sur le visage du porteur et présentant au moins une caractéristique géométrique prédéterminée.

Il est ensuite possible de déterminer le paramètre géométrico-physionomique recherché en comparant la caractéristique géométrique de l'image de l'élément de repère et sa caractéristique géométrique réelle correspondante.

Cependant, un tel procédé ne peut être mis en œuvre que si l'identification des éléments de repère sur l'image capturée est possible.

Ceci n'est notamment pas le cas si l'image capturée est floue ou si elle est surexposée ou sous-exposée au point que l'image des éléments de repère n'est pas identifiable.

En particulier, lorsqu'une image est capturée grâce à un dispositif comportant un moyen de réglage automatique de la distance focale d'acquisition de l'image, il est possible que le dispositif de capture d'image réalise une mise au point sur un point de l'image ne faisant pas partie de la tête du porteur, ce qui fait que la tête du porteur apparaît floue sur l'image capturée. En outre, lorsque le porteur est positionné en contre-jour par rapport au dispositif de capture d'image, l'image capturée est souvent trop sombre pour permettre l'identification de l'image de l'élément de repère.

Une solution à ces différents problèmes consiste à réaliser un réglage manuel de la netteté et de la luminosité de l'image capturée avant chaque capture d'image. Cependant, cela présente l'inconvénient d'être long et fastidieux pour l'opticien. En outre, cela n'est pas réalisable dans le cadre d'une acquisition d'image continue en mode vidéo. On connaît également des documents WO2010/119190 A1, JP2007289683 A et WO2010/145736 A1 des méthodes de détermination d'un paramètre géométrico-physionomique.

OBJET DE L'INVENTION

Afin de remédier aux inconvénients précités de l'état de la technique, la présente invention propose un procédé de détermination des paramètres géométrico-physionomiques d'un porteur dans sa position naturelle, quelles que soient les conditions de luminosité lors de la capture d'image et quelle que soit la position de la tête du porteur par rapport au dispositif de capture d'image.

Plus particulièrement, on propose selon l'invention un procédé de détermination, en posture naturelle, d'au moins un paramètre géométrico-physionomique associé au montage d'une lentille ophtalmique dans une monture de lunettes destinée à être portée par un porteur, tel que décrit dans la revendication 1

Ainsi, le fait que la tête du porteur soit dans une posture différente de la posture naturelle pendant la capture de l'image à l'étape a) est pris en compte lors de la détermination du paramètre recherché.

La différence entre la posture réelle du porteur lors de la capture d'image et la posture naturelle est évaluée par la comparaison entre la valeur réelle mesurée de l'angle d'inclinaison pendant la capture de l'image sensiblement de face et une valeur de référence de cet angle d'inclinaison correspondant à la posture naturelle du porteur.

On peut ensuite soit corriger la caractéristique géométrique mesurée sur l'image de l'élément de repère, soit corriger le paramètre géométrico-physionomique déterminé à partir de la caractéristique géométrique de l'élément de repère non corrigée pour obtenir la valeur du paramètre géométrico-physionomique recherché en posture naturelle.

D'autres caractéristiques non limitatives et avantageuses du procédé conforme à l'invention sont décrites dans les revendications 2 à 15. D'autres encore sont les suivantes :

- ledit angle d'inclinaison de la tête du porteur comporte une composante liée à la rotation de la tête du porteur autour d'un premier axe principal de la tête du porteur perpendiculaire au plan sagittal de la tête du porteur, qui correspond à une rotation de la tête du porteur par rapport à son corps, et/ou une composante liée à la rotation de la tête du porteur autour d'un deuxième axe principal perpendiculaire au plan sagittal de la tête du porteur, qui correspond à une rotation du corps du porteur autour de ce deuxième axe principal ;
- ledit élément de repère frontal est un élément anatomique remarquable de la tête du porteur ;
- ledit élément de repère frontal est situé sur un accessoire disposé sur la tête du porteur ;
- on réalise les étapes c1) successives grâce à un dispositif de capture d'image en mode vidéo ;
- ledit élément de repère de profil est un élément anatomique remarquable de la tête du porteur ;
- la position relative desdits éléments de repère frontal et de profil est prédéterminée et est utilisée pour déterminer le paramètre géométrico-physionomique recherché à l'étape d) ;
- à l'étape b), on mesure ladite valeur mesurée de l'angle d'inclinaison de la tête à l'aide d'un inclinomètre ;
- à l'étape c), on extrait la valeur de référence de l'angle d'inclinaison d'une base de données prédéterminée ;
- à l'étape c), on mesure la valeur de référence de l'angle d'inclinaison de la tête à l'aide d'un inclinomètre.

DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est un diagramme schématique des étapes d'un mode de réalisation possible de l'invention,
- la figure 2 est une vue schématique d'une image sensiblement de face capturée par le dispositif de capture d'image,
- la figure 3 est une vue schématique d'une image sensiblement de profil capturée par le dispositif de capture d'image,
- la figure 4 est une vue schématique de la tête du porteur équipée de la monture de lunettes et de l'accessoire,
- la figure 5 est une vue schématique en perspective d'un accessoire comportant des éléments de repère et de calibrage visibles de face et de profil pour la mise en œuvre du procédé selon l'invention,
- la figure 6A est une vue schématique de face d'un élément de repère et de calibrage de l'accessoire de la figure 5, visible sur une vue de profil de cet accessoire, et
- la figure 6B est une vue schématique de face d'un élément de repère et de calibrage de l'accessoire de la figure 5, visible sur une vue de face de cet accessoire,
- la figure 7 est une vue schématique de profil du porteur pendant la capture d'image à l'étape a).

On définit dans la suite la direction verticale comme celle donnée par un fil à plomb et la direction horizontale comme la direction perpendiculaire à cette direction verticale.

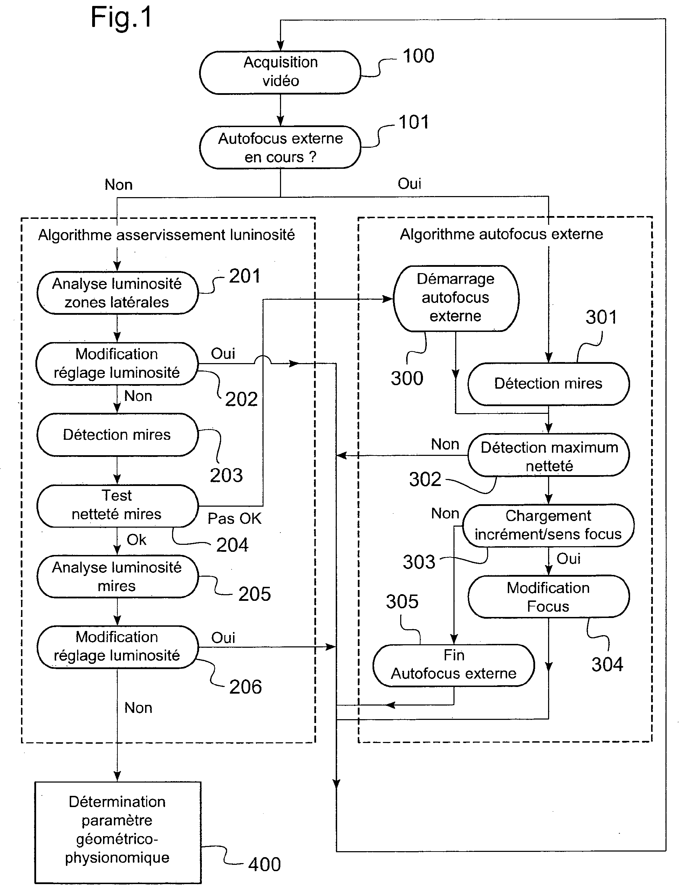

La figure 1 montre les étapes d'un mode de réalisation possible du procédé selon l'invention.

La mise en œuvre de ce procédé a pour but la détermination d'au moins un paramètre géométrico-physionomique associé au montage d'une lentille ophtalmique dans une monture de lunettes, dans la posture naturelle du porteur, tel que par exemple la distance interpupillaire, de la hauteur des pupilles des yeux du porteur par rapport au bord inférieur de la monture.

Le procédé comporte les étapes suivantes, qui sont décrites ci-après plus en détail:

a) on capture au moins une image sensiblement de face de la tête du porteur,

b) on détermine une valeur mesurée d'un angle d'inclinaison de la tête du porteur pendant la capture de l'image sensiblement de face, qui dépend de l'inclinaison de la tête du porteur autour d'un axe principal perpendiculaire à un plan sagittal de la tête du porteur,

c) on détermine une valeur de référence dudit angle d'inclinaison correspondant à une posture naturelle de la tête du porteur, et

d) on détermine ledit paramètre géométrico-physionomique recherché à partir de l'image sensiblement de face capturée et en fonction de la différence entre ladite valeur mesurée de l'angle d'inclinaison déterminé à l'étape b) et ladite valeur de référence dudit angle d'inclinaison déterminée à l'étape c).

Etape a)

On capture au moins une image sensiblement de face de la tête du porteur.

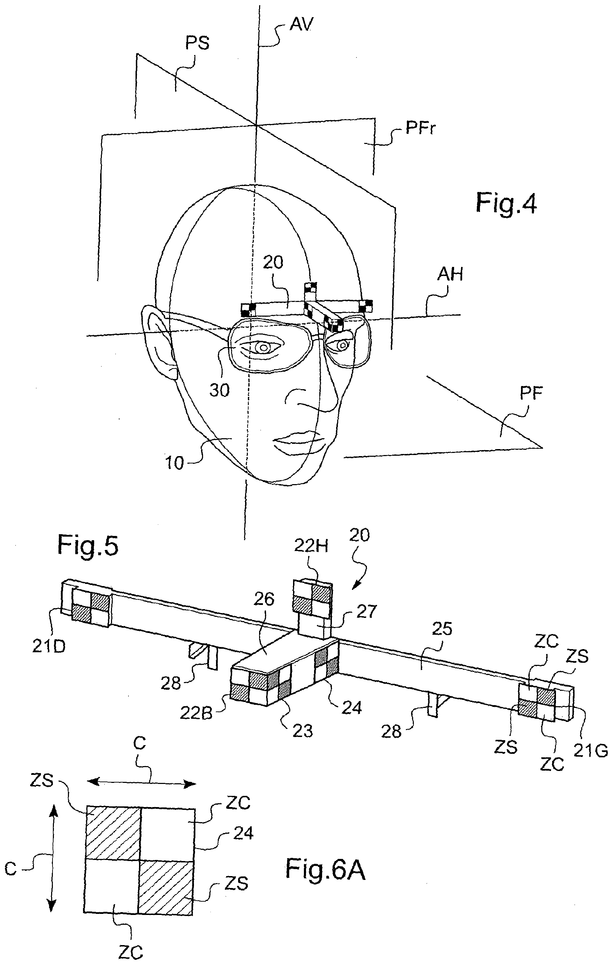

La figure 4 est une vue schématique de la tête 10 du porteur montrant la position de ses différents plans remarquables.

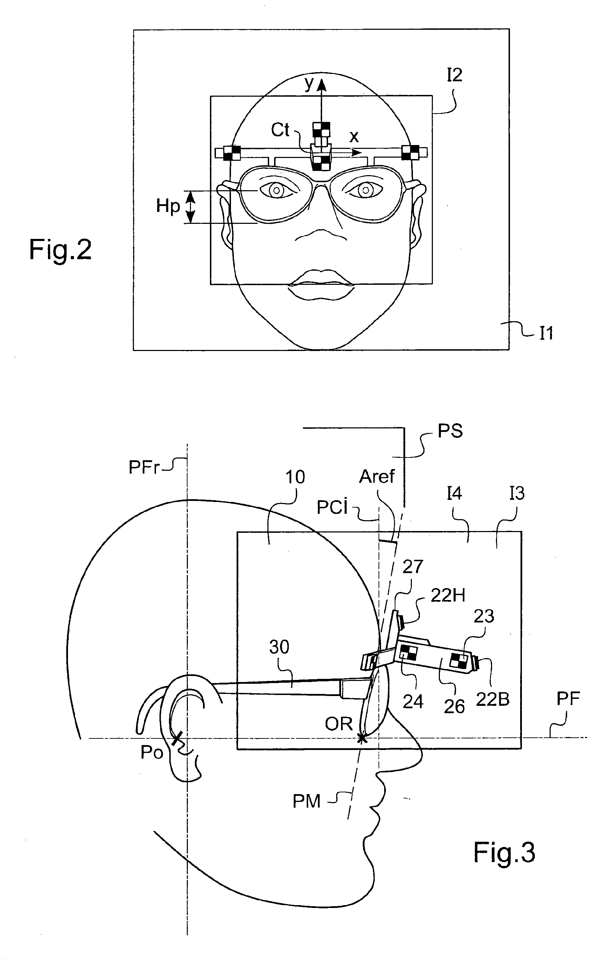

Comme représenté sur les figures 3 et 4, le plan de Francfort PF de la tête 10 du porteur est défini comme le plan passant par les points orbitaires inférieurs OR et le porion PO du porteur, le porion étant le point du crâne le plus élevé du conduit auditif, qui correspond au tragion de l'oreille.

Lorsque le porteur est en posture naturelle, ce plan de Francfort PF est sensiblement horizontal.

C'est le cas par exemple lorsque le porteur est dans une configuration assise ou debout dans laquelle sa tête 10 est droite et qu'il regarde droit devant lui, au loin, c'est-à-dire de préférence à l'horizon. L'axe de regard du porteur est alors horizontal.

On dit également que le porteur prend une position orthostatique, ou une position dans laquelle il réalise un minimum d'efforts.

On définit un plan sagittal PS de la tête 10 du porteur comme étant le plan perpendiculaire au plan de Francfort passant par la médiatrice des deux yeux. La médiatrice des yeux est l'axe passant au milieu du segment défini par les centres de rotation des deux yeux et parallèle au plan de Francfort PF.

On définit aussi un plan frontal PFr de la tête comme étant un plan perpendiculaire au plan de Francfort et passant par le sommet de la tête. Ce plan est également perpendiculaire au plan sagittal PS.

Dans ces conditions, un axe vertical AV de la tête 10 est défini comme l'intersection du plan frontal et du plan sagittal et un axe horizontal AH de la tête est défini comme l'intersection du plan de Francfort et du plan frontal.

Cet axe horizontal AH est donc un axe perpendiculaire au plan sagittal PS de la tête du porteur.

Une image sensiblement de face correspond alors à une image pour laquelle le plan de capture d'image du dispositif de capture d'image fait un angle compris entre +20 et - 20 degrés, autour de l'axe vertical AV avec le plan frontal PFr de la tête du porteur.

Une image sensiblement de profil correspond de manière similaire à une image pour laquelle le plan de capture d'image du dispositif de capture d'image fait un angle compris entre +10 et - 10 degrés autour de l'axe vertical AV avec le plan sagittal PS de la tête du porteur.

Un exemple d'image I1 sensiblement de face est montré à la figure 2. Un exemple d'image I3 sensiblement de profil est montré à la figure 3.

Etape b)

L'angle d'inclinaison A de la tête du porteur dont on détermine une valeur mesurée pendant la capture de l'image sensiblement de face dépend de l'inclinaison de la tête du porteur autour d'un axe principal perpendiculaire au plan sagittal de la tête du porteur.

Cet axe principal est par exemple ici l'axe horizontal AH. L'angle d'inclinaison mesuré correspond donc à un angle d'inclinaison dans le plan sagittal PS de la tête du porteur.

En pratique, on détermine par exemple la valeur mesurée de cet angle à partir de l'image sensiblement de face capturée à l'étape a).

On détermine par exemple la valeur mesurée de l'angle formé entre un plan solidaire de la tête 10 du porteur et un plan de capture d'image PCI associé à cette image capturée à l'étape a).

Le plan de capture d'image PCI correspond au plan de l'image capturée.

Le plan solidaire de la tête 10 du porteur est de préférence un plan perpendiculaire au plan sagittal PS de la tête du porteur.

Cet angle correspond plus précisément à l'angle entre l'intersection de ce plan solidaire de la tête du porteur et un plan perpendiculaire au plan de capture d'image et l'intersection du plan de capture d'image et de ce plan perpendiculaire audit plan de capture d'image.

Le plan solidaire de la tête 10 du porteur est par exemple le plan moyen des cercles de la monture 30 de lunettes portée par le porteur. En effet, lorsque la tête du porteur bouge, la monture de lunettes bouge simultanément et la position du plan moyen PM des cercles de la monture par rapport à la tête du porteur n'est pas modifiée.

En variante, le plan solidaire de la tête peut être un plan particulier de la tête elle-même, par exemple le plan frontal PFr. Il s'agit de préférence d'un plan parallèle à l'axe horizontal AH de la tête du porteur.

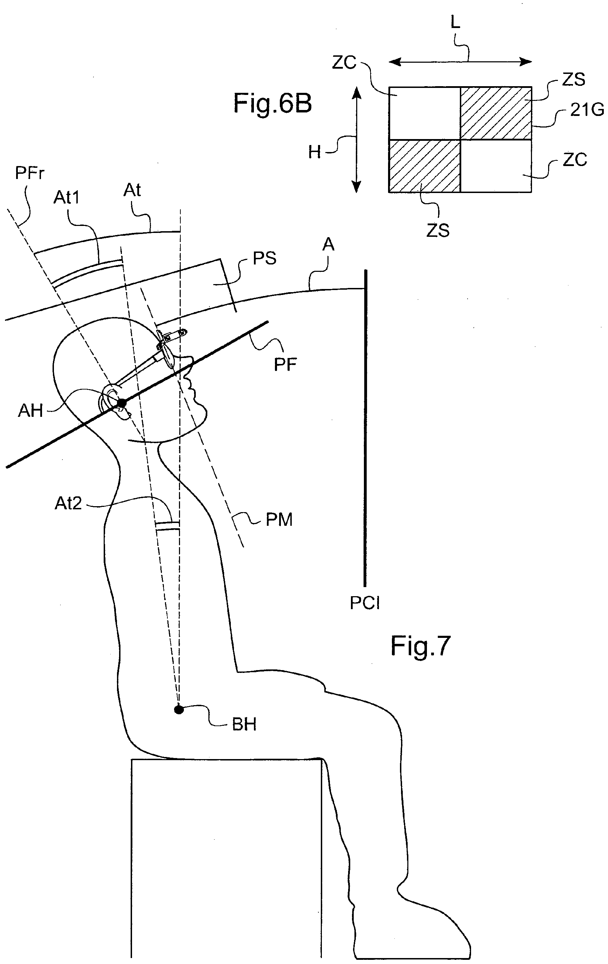

L'angle d'inclinaison A est représenté sur la figure 7. Le plan de capture d'image PCI est ici considéré comme orienté verticalement

Cet angle d'inclinaison A du plan moyen PM des cercles de la monture 30 varie de la même façon que l'angle At formé entre les intersections respectives du plan frontal PFr de la tête 10 du porteur et du plan de capture d'image PCI avec le plan perpendiculaire audit plan de capture d'image.

Ces deux angles A et At sont égaux à une constante près.

Comme représenté sur la figure 7, l'angle At du plan frontal PFr de la tête du porteur par rapport au plan de capture d'image PCI comporte ici une première composante At1 (voir figure 7) liée à la rotation du plan frontal PFr de la tête du porteur autour de l'axe horizontal AH de la tête du porteur correspondant à une rotation de la tête du porteur par rapport à son corps. L'angle At du plan frontal PFr de la tête du porteur par rapport au plan de capture d'image PCI comporte également une deuxième composante At2 (voir figure 7) liée à la rotation du plan frontal PFr de la tête 10 du porteur par rapport à un axe horizontal BH également perpendiculaire au plan sagittal PS de la tête du porteur. Cette deuxième composante correspond à une rotation du corps du porteur autour de cet axe horizontal BH.

On a représenté sur la figure 7 les deux composantes At1, At2 de l'angle At du plan frontal PFr par souci de simplification. Il est évident que l'angle d'inclinaison A du plan moyen PM des cercles de la monture du porteur comporte également deux composantes similaires, puisque l'inclinaison du plan moyen PM des cercles de la monture suit celle de la tête.

La tête 10 du porteur comporte de préférence au moins un élément de repère frontal disposé de telle sorte qu'il soit identifiable sur ladite image sensiblement de face du visage du porteur capturée à l'étape a).

Cet élément de repère frontal peut être un élément anatomique remarquable de la tête du porteur, par exemple la pupille et/ou l'iris de l'un des yeux, le contour de l'œil, les ailes du nez du porteur.

Cependant, l'élément de repère frontal est situé de préférence sur un accessoire disposé sur la tête du porteur.

Il peut s'agir par exemple d'un autocollant collé sur la tête du porteur.

Il s'agit de préférence d'un accessoire 20 tel que représenté sur la figure 5 et destiné à être monté sur la monture 30 de lunettes du porteur.

L'accessoire 20 comporte (figure 5) une barre principale 25 adaptée à être disposée au dessus de la monture de lunettes, dans le plan moyen PM des cercles de cette monture.

L'accessoire 20 dispose à cet effet de moyens de montage sur la monture 30 se présentant ici sous la forme de clips 28 s'étendant à partir de la barre principale 25.

Il est ici prévu deux clips 28, adaptés chacun à s'accrocher sur l'un des cercles de la monture 30.

Cet accessoire 20 comporte également une licorne 26 s'étendant perpendiculairement à la barre principale 25, dans un plan sensiblement perpendiculaire au plan moyen PM des cercles de la monture lorsque l'accessoire 20 est fixé sur cette monture 30, et un élément en saillie 27 s'élevant perpendiculairement à la barre principale 25 et à la licorne 26, dans le plan moyen PM de la monture 30 ou dans un plan parallèle à ce plan moyen PM lorsque l'accessoire 20 est fixé sur cette monture (figure 3).

Ici, l'accessoire 20 comporte huit éléments de repère 21D, 21 G, 22H, 22B, 23, 24. Deux éléments de repère 21D, 21G sont disposés aux extrémités de la barre principale 25, et sont orientés de manière à être visibles sur une image de face du porteur, lorsque l'accessoire 20 est fixé sur la monture du porteur. Ces éléments de repère sont donc des éléments de repère frontaux de la tête du porteur.

Un élément de repère 22H est disposé sur l'élément en saillie 27 et un autre élément de repère 22B est disposé à l'extrémité de la licorne 26, de telle sorte que ces deux éléments de repère 22H, 22B sont visibles sur une image de face du porteur. Ces éléments de repère sont donc également des éléments de repère frontaux de la tête du porteur.

En outre, ces deux éléments de repère 22H, 22B sont disposés de telle sorte que, sur une image de face de l'accessoire, ils sont situés l'un en dessous de l'autre.

Enfin les côtés latéraux de la licorne portent également chacun deux éléments de repère 23, 24 qui sont visibles sur les images sensiblement de profil du porteur, comme expliqué plus en détail ultérieurement. Ces éléments de repère sont donc des éléments de repère de profil de la tête du porteur.

Chaque élément de repère de face ou de profil présente une ou plusieurs caractéristiques géométriques prédéterminées, par exemple ses dimensions ou les dimensions d'un motif géométrique porté par lui. Le motif géométrique peut par exemple se présenter sous la forme d'une mire ou de bandes contrastées alternées.

Chaque élément de repère comporte ici quatre zones contrastées ZS, ZC. Ces zones contrastées sont disposées en alternance, chaque zone formant un angle droit de sommet commun avec les angles droits formés par les autres zones. Ledit sommet commun des zones claires et sombres forme le centre de l'élément de repère. Ces éléments de repère sont aussi appelés « mires », comme cela est le cas sur la figure 1 par exemple.

En pratique, comme représenté plus en détail sur la figure 6B pour l'élément de repère frontal 21G, chaque élément de repère frontal 21D, 21G, 22H, 22B, se présente ici sous la forme d'un rectangle de longueur L comprise entre 8 et 11 millimètres et de hauteur H comprise entre 5 et 8 millimètres.

Ce rectangle est divisé en quatre rectangles plus petits de dimensions égales. Les plus petits rectangles présentent deux à deux en diagonal des luminances ou des couleurs identiques, et présentent deux à deux avec leur voisin des luminances ou des couleurs différentes.

En variante, lesdits éléments de repère peuvent présenter toute autre forme, notamment une forme carré ou circulaire.

Comme représenté plus en détail sur la figure 6A pour l'élément de repère de profil 24, les éléments de repère de profil 23, 24 présentent de préférence une forme carrée avec un côté de longueur Ct égal à 6 millimètres. Ce carré est divisé en quatre carrés plus petits de dimensions égales. Les plus petits carrés présentent deux à deux en diagonal des luminances ou des couleurs identiques, et présentent deux à deux avec leur voisin des luminances ou des couleurs différentes.

En variant, les éléments de repère de face et de profil peuvent ne présenter que deux ou trois zones contrastées, par exemple un disque sombre concentrique avec un disque plus clair ou inversement, ou deux zones contrastées séparées par une ligne droite, ou encore une bande claire entre deux bandes sombres par exemple. On peut au contraire prévoir plus de quatre zones contrastées, par exemple des anneaux contrastés concentriques ou une alternance de bandes claires et sombres.

Afin de déterminer la valeur mesurée de l'angle d'inclinaison A, on réalise par exemple les sous-étapes suivantes :

b1) on identifie sur l'image sensiblement de face capturée à l'étape a) l'image de l'élément de repère frontal,
b2) on détermine une caractéristique géométrique de l'image de cet élément de repère frontal, et
b3) on détermine ledit angle d'inclinaison A associé à l'image sensiblement de face en fonction de cette caractéristique géométrique.

Les étapes b1) et b2) peuvent être réalisées manuellement par l'opérateur ou automatiquement par un traitement de l'image capturée à l'étape a).

Un exemple de traitement de l'image capturée à l'étape a) permettant d'identifier l'image de chaque élément de repère frontal à l'étape b1) sur l'image sensiblement de face sera donné ultérieurement.

On peut déduire de la position des images des éléments de repère la position d'autres points remarquables de l'image.

En particulier la position d'un point Ct situé au milieu des centres des éléments de repère 21D, 21G disposés à chaque extrémité de la barre 25 de l'accessoire 20 (figure 2).

Selon un exemple de traitement possible, à l'étape b2), on détermine les coordonnées des centres des images des éléments de repère sur l'image capturée, dans un référentiel de l'image.

En particulier, on détermine les coordonnées du centre de l'élément de repère frontal 22B situé à l'extrémité de la licorne de l'accessoire dans ce référentiel et celles du point Ct défini précédemment. On en déduit la longueur du segment reliant le centre de l'image de l'élément de repère 22B situé à l'extrémité de la licorne et le point Ct sur l'image.

Dans un cas particulier où l'image capturée à l'étape a) est capturée à un instant où l'axe horizontal AH de la tête est parallèle au plan de capture d'image PCI, on définit de préférence le référentiel de l'image Ct(x,y) qui a pour origine le point Ct et des axes x et y s'étendant respectivement le long de l'image de la barre 25 de l'accessoire et de l'image de l'élément de saillie 27 de l'accessoire, comme représenté sur la figure 2.

Dans ces conditions, la longueur du segment recherchée est égale à la coordonnée yb selon l'axe y du centre de l'élément de repère 22B.

La distance Db existant en réalité entre l'élément de repère 22B situé sur la licorne et le point Ct de l'accessoire 20 est par ailleurs connue par construction de l'accessoire 20.

La distance Db existant entre l'élément de repère 22B situé sur la licorne et le point Ct de de l'accessoire 20 est connue par construction.

On en déduit alors la valeur mesurée Am de l'angle d'inclinaison A sur l'image par la formule : Am = arcsin(yb / Db), où arcsin est la fonction réciproque de la fonction sinus.

En variante, à l'étape b), on mesure ladite valeur mesurée de l'angle d'inclinaison de la tête à l'aide d'un inclinomètre

Etape c)

On détermine une valeur de référence de l'angle d'inclinaison qui correspond à la valeur de cet angle lorsque la tête du porteur est dans une posture naturelle.

La valeur de référence Aref de l'angle d'inclinaison A peut par exemple être déterminée à l'étape c), par la réalisation des étapes suivantes :

c1) on capture au moins une image sensiblement de profil de la tête 10 du porteur en posture naturelle, la tête 10 du porteur comportant au moins un élément de repère de profil disposé de telle sorte qu'il soit identifiable sur ladite image sensiblement de profil du visage du porteur,

c2) on identifie, sur ladite image sensiblement de profil, l'image de cet élément de repère de profil,

c3) on détermine une caractéristique géométrique de l'image de cet élément de repère de profil,

c4) on détermine la valeur de référence Aref dudit angle d'inclinaison A en fonction de la caractéristique géométrique de l'image déterminée à l'étape c3).

Une telle image de profil est représentée sur la figure 3.

Sur cette figure 3, le porteur est dans la posture naturelle. Le plan de Francfort est horizontal et l'angle d'inclinaison entre le plan moyen PM des cercles de la monture de lunettes 30 du porteur et le plan de capture d'image PCI correspondant à la capture d'image de l'étape a) est alors égal à la valeur de référence recherchée.

On peut, pour réaliser cette capture d'image de profil, soit demander au porteur de tourner la tête d'environ 90 degrés, soit déplacer le dispositif de capture d'image de telle sorte que le plan de capture d'image pivote de 90 degrés autour d'un axe vertical.

Dans ce dernier cas, le dispositif de capture d'image étant déplacé à l'étape c), il est nécessaire de connaître sa position et son orientation à l'étape c) par rapport à la position et l'orientation du dispositif de capture d'image pendant la capture d'image sensiblement de face de l'étape a).

On a décrit ici un exemple dans lequel le plan de capture d'image facial à l'étape a) est sensiblement vertical.

Bien entendu, ce plan de capture d'image facial peut être non vertical.

Dans un cas plus général, on prévoit des moyens de détermination de l'orientation dans un référentiel de l'espace du dispositif afin de déterminer l'orientation du dispositif par rapport à deux axes horizontaux perpendiculaires. Ces moyens permettent ainsi la détermination d'un angle de tangage et de roulis relatifs à chacun de ces deux axes.

De tels moyens sont connus par exemple du document US12596351.

Il est prévu ici deux éléments de repère de profil 23, 24 sur l'accessoire 20, comme décrit précédemment.

A l'étape c1), le porteur est placé en position naturelle. Pour cela, qu'on lui demande de pivoter la tête par rapport au dispositif de capture d'image, ou que l'on pivote le dispositif de capture d'image par rapport à lui, il est assis ou debout, tête droite, et regarde au loin, selon une direction approximativement perpendiculaire à l'axe optique du dispositif de capture d'image.

Le porteur regarde par exemple à l'horizon si cela est possible. En variante, il regarde un point situé à plus de deux mètres de lui, de préférence situé à plus de 5 mètres droit devant lui.

Ce point peut être matérialisé par une cible sur un mur situé devant le porteur.

En outre, comme mentionné précédemment, dans une image sensiblement de profil le plan de capture d'image PCI du dispositif de capture d'image fait un angle compris entre +10 et - 10 degrés autour de l'axe AV avec le plan sagittal PS de la tête du porteur. De préférence, le plan sagittal PS de la tête du porteur est parallèle au plan de capture d'image.

A l'étape c2), on identifie, sur ladite image sensiblement de profil, l'image de chaque élément de repère de profil 23, 24. Le traitement de l'image de profil réalisé pour identifier ces éléments de repère de profil sera détaillé ultérieurement.

A l'étape c2), on détermine les coordonnées, dans un repère du plan de l'image par exemple attaché à un coin de cette image, des éléments de repère de profil 23, 24, par exemple, on détermine les coordonnées de leurs centres, puis les coordonnées (X, Y) du segment reliant leur centre.

La valeur de référence Aref de l'angle d'inclinaison A entre le plan moyen des montures PM et le plan de capture d'image PCI à l'étape a) est alors égale à arctan(Y/X). De préférence, le dispositif de capture d'image est une caméra vidéo et on capture une série d'image sensiblement de profil de la tête du porteur.

Le dispositif de capture d'image vidéo capture de préférence entre 1 et 30 images par seconde, de préférence entre 5 et 20 images par seconde, par exemple 15 images par secondes.

Toutes les images capturées ne sont pas utilisées pour déterminer le paramètre géométrico-physionomique recherché, mais certaines d'entre elles permettent d'affiner les réglages du dispositif de capture d'image, comme expliqué ci-après, de manière à améliorer la luminosité et la netteté des images capturées ultérieurement des éléments de repère de l'accessoire 20, de manière à assurer leur identification précise.

On répète alors les étapes c2) à c4) pour une pluralité d'images de la série et on détermine la valeur de référence Aref de l'angle d'inclinaison A comme la valeur moyenne des valeurs de référence déterminées pour chaque image.

La valeur de référence obtenue est alors plus précise.

De préférence, on calcule une première moyenne arithmétique de toutes les valeurs de référence déterminées à partir de la série d'images et on détermine un écart type de chaque valeur de référence par rapport à cette première moyenne.

On élimine ensuite de l'ensemble des valeurs de référence considérées celles dont l'écart avec la première moyenne calculée est supérieur à un écart seuil déterminé en fonction de l'écart type. L'écart seuil est par exemple égal à deux fois l'écart type calculé.

On calcule ensuite la moyenne arithmétique des valeurs de référence restantes et on identifie cette deuxième moyenne calculée à la valeur moyenne de référence recherchée.

En variante, comme pour les éléments de repère de face, le ou les éléments de repère de profil peuvent être des éléments anatomiques remarquables de la tête du porteur.

En variante également, la valeur de référence de l'angle d'inclinaison peut être déterminée à l'étape c) par un inclinomètre.

En variante encore, cette valeur de référence peut être prédéterminée et mise en mémoire dans une table, pour un porteur donné. Elle peut ensuite être seulement extraite de cette base pour la mise en œuvre de l'étape c).

Etape d)

On détermine enfin le paramètre géométrico-physionomique recherché à partir de l'image sensiblement de face capturée à l'étape a) en fonction de la différence entre la valeur Am mesurée et la valeur de référence Aref de l'angle d'inclinaison A.

En pratique, on détermine le paramètre géométrico-physionomique soit en mesurant sur l'image capturée la grandeur correspondant au paramètre recherché dans le plan de capture d'image et en tenant compte d'un facteur d'échelle fonction de la distance entre la tête et le plan de capture d'image PCI et fonction de l'inclinaison de la tête par rapport à ce plan de capture d'image. On mesure par exemple la hauteur des pupilles par rapport au bord inférieur de la monture ou la distance interpupillaire, et on multiplie la grandeur mesurée par le facteur d'échelle.

Il est alors possible, soit de corriger la grandeur mesurée en fonction de la valeur de référence de l'angle d'inclinaison, soit de corriger le paramètre géométrico-physionomique déterminé en fonction de cette valeur de référence, afin d'obtenir le paramètre géométrico-physionomique correspondant à la posture naturelle du porteur.

Par exemple, dans le cas de la détermination de la hauteur Hp des pupilles des yeux, la distance correspondante, représentée sur la figure 2, est mesurée sur l'image sensiblement de face capturée à l'étape a). Cette distance peut être déterminée automatiquement par un traitement de l'image identifiant la position des images des reflets cornéens d'une source lumineuse placée à proximité du dispositif de capture d'image comme la position des pupilles et l'image du bord inférieur de la monture.

La valeur de référence de l'angle d'inclinaison est alors introduite dans le calcul classique de la hauteur des pupilles.

Plus précisément, la hauteur Hp des pupilles est par exemple déterminée à partir de l'image capturée sensiblement de face à l'étape a) selon une méthode connue décrite dans le document US12596351.

La hauteur des pupilles Hpn en posture naturelle est alors calculée en fonction de la distance dCRO connue entre le centre de rotation de l'œil et la lentille de la monture correspondante par la formule :

Hpn = Hp + dCRO * tan(Aref - Am) où tan est la fonction tangente et la convention d'angle est telle que les angles croissent lorsque le porteur penche la tête en avant.

Comme expliqué précédemment, les étapes b1) et c2) du procédé reposent respectivement sur l'identification de l'image d'au moins un élément de repère de face sur l'image sensiblement de face capturée à l'étape a) et sur l'identification de l'image d'au moins un élément de repère de profil sur l'image sensiblement de profil capturée à l'étape c1).

Afin d'assurer que l'identification de ces images soit possible, le procédé selon l'invention propose également d'assurer la capture d'une image de face et/ou de profil exploitable pour le traitement d'image devant être réalisé lors des étapes précitées.

A cet effet, il convient de s'assurer de préférence que deux conditions simultanées sont remplies pour chaque image capturée, à savoir que la luminosité d'au moins une partie de l'image contenant l'image des éléments de repère est suffisante, et que cette partie de l'image est nette.

Ces deux réglages sont particulièrement importants dans le cas de la capture d'image de profil et seront décrits ci-après dans ce cadre.

Ils s'appliquent néanmoins de la même façon aux images capturées de face.

Etapes préliminaires : réglage de la luminosité et/ ou réglage de netteté de l'image

*Réglage grossier de la luminosité*

On peut prévoir de réaliser tout d'abord un premier réglage grossier et rapide des paramètres d'acquisition du dispositif de capture d'image, par les étapes suivantes, représentées sur les blocs 100, 201, 202 de la figure 1 :

> p1) on capture une image préliminaire sensiblement de profil de la tête du porteur (bloc 100),
> p2) on détermine une valeur mesurée de la luminosité moyenne d'au moins une zone réduite de cette image préliminaire, cette zone réduite étant adaptée à couvrir au moins une partie de l'image de la tête du porteur (bloc 201),
> p3) on ajuste grossièrement le paramètre de réglage des conditions optiques d'acquisition du dispositif de capture d'image en fonction de cette valeur mesurée de manière à améliorer le contraste de l'image de la tête du porteur capturée (bloc 202),
> p4) on utilise le paramètre de réglage des conditions optiques d'acquisition obtenu à l'étape p3) pour ajuster les conditions optiques des captures d'images ultérieures, notamment à l'étape c1) (retour au bloc 100).

La zone réduite de l'image capturée à l'étape p1) utilisée à l'étape p2) est par exemple une zone latérale gauche ou droite de l'image selon que la licorne de l'accessoire se situe à gauche ou à droite de l'image de profil. Il s'agit par exemple sur l'image I3 de la figure 3 de la moitié droite I4 de cette image.

Cette zone réduite est par exemple une zone centrale I2 (figure 2) de l'image I1 lorsque l'image est capturée de face.

On détermine à l'étape p2) une luminance ou une chrominance moyenne de cette zone réduite de l'image et, à l'étape p3) :

- on détermine l'écart entre la valeur mesurée déterminée à l'étape p2) et une valeur cible de luminosité,
- on compare cet écart avec une valeur maximale prédéterminée de cet écart, et

en fonction de cette comparaison, à l'étape p3), on ajuste le paramètre de réglage des conditions optiques d'acquisition du dispositif de capture d'image de manière à faire tendre ladite valeur mesurée vers ladite valeur cible de luminosité. Cet ajustement est détaillé ci-après dans la partie relative au réglage fin de la luminosité.

Si le paramètre de réglage des conditions optiques d'acquisition du dispositif de capture d'image a été modifié, on répète les étapes p1) à p4) (bloc 100, 201, 202).

Si la luminosité moyenne sur la zone latérale de l'image évaluée à l'étape p2) est satisfaisante, c'est-à-dire que l'écart entre la valeur mesurée déterminée à l'étape p2) et une valeur cible de luminosité est inférieure à la valeur maximale de l'écart prédéterminée, on peut ensuite réaliser un réglage de la netteté de l'image et un réglage fin de la luminosité.

L'étape de réglage grossier de la luminosité est optionnelle et on peut également réaliser directement les réglages de netteté et de luminosité exposés ci-après.

Les premières étapes de ces deux réglages consistent en une capture d'une image sensiblement de profil de la tête du porteur (bloc 100 de la figure 1), et en une détection de la position des éléments de repère (bloc 203 et 301 de la figure), qui forment ici des éléments de calibrage de la luminosité et des éléments d'évaluation de la netteté.

*Identification des éléments de repère : détermination de leur position*

En pratique, on capture une série d'images à intervalles de temps réguliers (bloc 100 de la figure 1). Les paramètres de capture d'image du dispositif de capture sont modifiés selon les étapes décrites ci-après au fur et à mesure de la capture des images et de leur traitement.

Les images capturées sont ici par exemple des images noir et blanc dans lesquelles chaque pixel présente une luminance donnée.

En variante, on peut également envisager de capturer une image en couleur, c'est-à-dire dans laquelle chaque pixel contient une information de luminance et de chrominance, et convertir cette image en couleur en une image en tons de gris.

Pour cela, l'image capturée en codage (R,G,B) comportant des composantes rouge R, verte G, et bleu B est transformée de manière connue en coordonnées (Y,U,V) comportant des composantes de luminance Y et chrominance U,V. La luminance de chaque pixel est obtenue selon la formule :

$$Y = 0,299*R + 0,587*G + 0,114*B.$$

L'image de chaque élément de repère présente donc sur l'image capturée un agencement de zones claires et sombres correspondant aux images des zones claires et sombres de l'élément de repère correspondant, c'est-à-dire une répartition de luminosité déterminée. Cette répartition de luminosité peut être une répartition de luminance ou de chrominance.

On réalise de préférence tout d'abord une étape de ré-échantillonnage de l'image capturée, destinée à réduire le nombre de pixels total de l'image.

Les étapes ultérieures de traitement de l'image décrites ci-dessous sont alors plus rapides, car les temps de calcul sont réduits avec la réduction du nombre de pixels de l'image.

Le coefficient de ré-échantillonnage est par exemple compris entre 1,5 et 3. Il est par exemple égal à 2.

En variante, il est possible d'utiliser l'image capturée non ré-échantillonnée.

Pour déterminer la position de l'image de chaque élément de repère sur l'image capturée, on réalise une étape s) de convolution d'au moins une partie de l'image capturée par une matrice de détection reproduisant la répartition de luminosité attendue de l'image de l'un des éléments de repère.

Ici, les deux éléments de repère de profil 23, 24 ne sont pas identifiés lors du traitement d'image décrit car ils présentent une répartition de luminance différente de celle des éléments de calibrage de face 21D, 21G, 22H, 22B.

En pratique ici les zones claires et sombres sont inversées sur les éléments de repère de profil. La matrice de détection utilisée pour déterminer la position des éléments de repère frontaux n'est donc pas adaptée pour déterminer les éléments de repère de profil. Cette répartition de luminosité peut être une répartition de luminance ou de chrominance selon le type d'élément de repère utilisé, comme expliqué précédemment.

Ici, étant donnée l'agencement des zones sombres ZS et claires ZC de chaque élément de repère de profil 23, 24, à l'étape s) de convolution, ladite matrice de détection est de la forme :

$$\begin{vmatrix} m & -m \\ -m & m \end{vmatrix}$$

Chaque élément m de la matrice de détection est une sous-matrice comportant un nombre de lignes et un nombre de colonnes tels que la matrice de convolution présente des dimensions inférieures ou égales aux dimensions correspondantes en pixels de l'image de l'un des éléments de repère.

Par exemple, dans le cas des éléments de repère de profil, si l'élément de repère mesure en réalité 6 millimètres de long sur 6 millimètres de hauteur et que son image s'étend initialement sur l'image capturée sur 12 pixels en longueur et 12 pixels en hauteur, après ré-échantillonnage, elle s'étend sur 6 pixels en longueur et 6 pixels en hauteur.

Chaque zone sombre ZS et chaque zone claire ZC de l'élément de repère s'étend donc sur 3 pixels en longueur et 3 pixels en hauteur.

Chaque élément m de la matrice de détection comporte alors par exemple 3 colonnes et 3 lignes.

Dans l'exemple décrit ici, la luminosité de chaque zone sombre ou claire est uniforme, les coefficients de chaque élément m de la matrice de détection sont alors de préférence tous égaux.

En outre, la somme des coefficients de chaque élément m de la matrice de détection est de préférence égale à 1/2 afin d'éviter des phénomènes de saturation lors de la convolution.

Dans l'exemple présenté ici, la matrice m se présente sous la forme :

$$m = 1/18* \begin{vmatrix} 1 & 1 & 1 \\ 1 & 1 & 1 \\ 1 & 1 & 1 \end{vmatrix}$$

La convolution de l'image capturée par cette matrice de détection est par exemple réalisée pour la totalité de l'image. Ceci permet d'identifier l'image de chaque élément de repère, sans connaître a priori la position de celui-ci dans l'image, ce qui est notamment le cas lors de la convolution de la première image d'une série d'images.

On pourrait cependant également envisager de réaliser avant cette étape de convolution, une étape d'estimation approximative de la position des éléments de repère.

Il est également possible d'effectuer un suivi de la position des éléments de repère : à partir de la position d'un élément de repère sur une image donnée de la tête du porteur, une position approximative de cet élément de repère sur l'image suivante peut être estimée, par exemple en fonction de l'intervalle de temps entre deux captures d'image et de la vitesse moyenne de déplacement de la tête d'un porteur.

Seule une partie de l'image capturée suivante, située dans la région déterminée précédemment est alors convoluée par la matrice de détection. Le calcul est ainsi plus rapide.

Dans l'exemple décrit, la région de l'image convoluée par la matrice de détection comporte des groupes de pixels appartenant aux éléments de repère et d'autres groupes de pixels qui n'appartiennent pas aux éléments de repère.

Il est alors possible d'effectuer une étape t) de renforcement du contraste de l'image convoluée à l'étape s), au cours de laquelle on augmente la luminance des groupes de pixels appartenant aux éléments de repère. Pour cela, on convolue au moins une partie de l'image capturée par une matrice de renforcement dont les dimensions sont inférieures ou égales aux dimensions correspondantes en pixels de l'image d'un élément de repère et dont les coefficients augmentent des bords vers le centre de la matrice de renforcement.

La taille de cette matrice de renforcement dépend de la taille de l'image de l'élément de calibrage, et donc du coefficient de ré-échantillonnage.

La convolution de l'image obtenue à l'étape s) par cette matrice de renforcement permet d'augmenter la luminance des pixels situés dans les zones dont la luminance suit la répartition attendue pour l'image d'un élément de repère afin de faciliter leur détection.

La détermination de la position des éléments de repère est ainsi plus précise.

On effectue ensuite, sur l'image obtenue à l'étape t), ou sur l'image obtenue à l'étape s) si la convolution par la matrice de renforcement de contraste n'est pas réalisée, une étape u) de recherche des maxima isolés.

Plus précisément, à l'étape u), on détecte des groupes de pixels présentant un pic de luminosité isolé et présentant une taille inférieure à la taille de l'élément de calibrage de luminosité dans toutes les directions.

Pour cela, on repère sur l'image obtenue à l'étape s) ou t) les pixels dont la luminosité est supérieure à une valeur seuil de luminosité prédéterminée.

Il peut bien entendu s'agir d'une valeur seuil de luminance ou de chrominance selon le type d'élément de repère utilisé.

On utilise ensuite un algorithme de remplissage d'une matrice d'isolement centrée sur chaque pixel repéré. Cet algorithme remplit la matrice avec les valeurs de luminosité des pixels voisins dudit pixel repéré si cette luminosité est supérieure à ladite valeur seuil.

La taille de la matrice d'isolement est choisie en fonction de la taille de l'image recherchée de l'élément de repère.

Si la matrice d'isolement reste vide le long de ses bords, ceci signifie que le groupe de pixel situé autour du pixel repéré correspond à un maximum isolé.

On effectue par exemple une première sélection parmi tous les groupes de pixels identifiés à l'étape u) en ne retenant que les dix groupes dont la luminosité est la plus grande.

On réalise ensuite une étape v) de sélection d'au moins deux des groupes de pixels qui présentent un pic de luminosité isolé détectés à l'étape u) et présentant la plus forte probabilité d'être associés à l'image de deux des éléments de repère de l'accessoire, à savoir au moins deux groupes de pixels pour lesquels est réduit l'écart entre :

- une distance mesurée entre ces deux groupes et une distance de référence, et/ou
- un angle mesuré entre la droite passant par ces deux groupes et une direction de référence et un angle de référence, et/ou
- la luminosité mesurée au voisinage de ces deux groupes et une luminosité de référence, et/ou
- la différence de luminosité entre deux points de positions relatives prédéterminées par rapport à chaque groupe de pixels et une différence de luminosité de référence.

Pour cela, on détermine tous les couples de groupes de pixels présents sur l'image selon une direction donnée, par exemple en considérant un premier groupe de pixels et chaque autre groupe de pixels situé à gauche ou à droite de ce premier groupe, ou au-dessus ou en-dessous de ce premier groupe.

On attribue une note globale à chaque couple de groupes de pixels de manière à quantifier leur ressemblance avec l'image d'un couple d'élément de repère donné de l'accessoire.

La note globale attribuée à chaque couple est déterminée en fonction de la comparaison de caractéristiques géométriques et de luminosité des groupes de pixels avec les caractéristiques attendues de l'image du couple d'éléments de repère considéré.

Par exemple, on compare la distance mesurée sur l'image entre les deux groupes de ce couple et la distance attendue entre les images des deux éléments de repère 23, 24, et on attribue une première note intermédiaire aux couples considérés qui est d'autant plus grande que l'écart entre ces deux distances est faible. La distance attendue entre les images des deux éléments de repère est déterminée en fonction d'un facteur d'échelle prenant notamment en compte le ré-échantillonnage de l'image.

On compare également l'angle mesuré sur l'image entre la droite passant par ces deux groupes et une direction représentant par exemple l'image d'une droite horizontale et l'angle attendu entre la droite reliant les images des deux éléments de repère et l'image d'un plan horizontal. Une deuxième note intermédiaire d'autant plus grande que l'écart entre ces deux angles est petit est alors attribuée au couple considéré.

On compare également la luminosité mesurée au voisinage de ces deux groupes et une luminosité de référence déterminée en fonction des captures d'image précédentes.

Lorsqu'une série d'images est capturée, on peut supposer que les valeurs de luminosité mesurées sur deux images successives seront proches. Ainsi, on attribue au couple considéré une troisième note intermédiaire d'autant plus grande que l'écart de luminosité précité est petit.

Enfin, on peut ici par exemple comparer, pour chaque groupe de pixels dudit couple, une différence de luminosité de référence et la différence de luminosité entre deux points de positions relatives choisies pour qu'ils soient situés dans deux cadrans voisins et/ou dans deux cadrans diagonalement opposés de l'image de l'élément de repère dans le cas où ce groupe de pixels serait l'image d'un élément de repère.

On peut également exclure de la sélection réalisée à l'étape v) les couples de deux groupes de pixels pour lesquels l'écart entre une distance mesurée entre ces deux groupes et une distance de référence, et/ou un angle mesuré entre la droite passant par ces deux groupes et une direction de référence et un angle de référence, et/ou la luminosité mesurée au voisinage de ces deux groupes et une luminosité de référence et/ou la différence de luminosité entre deux points de positions relatives par rapport à chaque groupe de pixels prédéterminés et une différence de luminosité de référence est supérieur à une valeur seuil.

La note globale de chaque couple est par exemple obtenue en multipliant toutes les notes intermédiaires attribuées à ce couple. En variante, ces notes intermédiaires peuvent être pondérées selon l'importance ou la fiabilité du critère considéré.

Le couple de groupes de pixels présentant la meilleure note globale est identifié au couple d'images des éléments de repère recherché.

Dans le cas où cette détermination des positions des images des éléments de repère est réalisée sur une image ré-échantillonnée, on peut envisager de répéter sur l'image non ré-échantillonnée l'étape de convolution par une matrice de détection reproduisant la répartition de luminosité de l'image attendue de l'élément de repère, dans une zone réduite de l'image non-ré-échantillonnée centrée autour de la position de l'image non. ré-échantillonnée correspondant à chaque position déterminée pour les éléments de repère sur l'image ré-échantillonnée.

La taille de la matrice de détection utilisée dans cette étape est alors adaptée à l'image non ré-échantillonnée.

*Réglage de la netteté*

Ce réglage peut être réalisé après ou avant le réglage grossier et/ou fin de la luminosité.

Dans l'exemple de la figure 1, il est réalisé après le réglage grossier de la luminosité et avant son réglage fin.

Ce réglage comporte, après une étape h) de capture d'une image sensiblement de profil de la tête du porteur, et une étape i) de détermination de la position de l'image d'au moins l'un des éléments de repère de profil sur cette image capturée à l'étape h), selon le procédé décrit précédemment, les étapes suivantes :

j) on détermine un paramètre de netteté représentatif de la netteté de l'image de cet élément de repère de profil sur cette image,

k) on compare ce paramètre de netteté avec une valeur seuil de netteté prédéterminée,

l) on modifie la distance focale d'acquisition d'image dudit dispositif de capture d'image en fonction de la différence entre le paramètre de netteté déterminé à l'étape j) et ladite valeur seuil de netteté, de manière à améliorer la netteté de l'image de l'élément de repère de profil.

A l'étape j), on peut utiliser l'image capturée à l'étape h) ou l'image convoluée par la matrice de détection obtenue à l'étape i) lors de la détermination de la position de l'image de l'élément de repère.

Les étapes j) et k) forment un test de netteté représenté par le bloc 204 sur la figure 1.

A l'étape j), on détermine la taille ou la luminosité de l'image d'un point particulier de l'élément de repère de profil et on en déduit ledit paramètre de netteté.

Par exemple, on détermine la taille et la forme de la tâche représentant le centre de l'élément de repère selon la position de cet élément de repère déterminée à l'étape précédente.

Plus cette taille est grande, plus l'image est floue.

De même, plus l'image est floue, et plus la luminosité du centre de l'élément de repère est faible.

Ainsi, si la taille de la tâche représentant le centre de l'élément de repère est supérieure à une valeur seuil de taille ou si la luminosité de cette tâche est inférieure à 50% du pic de luminance, la netteté de l'image n'est pas satisfaisante et on déclenche à l'étape l) un programme d'ajustement de la distance focale d'acquisition du dispositif de capture d'image (bloc 300 de la figure 1).

Ce programme comporte les étapes suivantes : initialisation (bloc 300), détection de la distance focale correspondant à une netteté maximum des éléments de repère (bloc 302), changement de l'incrément et du sens de modification de la distance focale (bloc 303), ajustement de la distance focale d'acquisition (bloc 304).

La distance focale d'acquisition est par exemple réglable entre 50 et 70 centimètres.

Lors du démarrage de l'algorithme, les initialisations suivantes sont effectuées :

- la distance focale du dispositif de capture d'image est réglée à sa valeur minimale, appelée FocusMin, égale à 50 centimètres dans l'exemple,

- l'incrément de la distance focale du dispositif de capture d'image, appelé DeltaFocus et égal à l'écart entre deux valeurs successives de la distance focale testée par le programme, est réglé à une valeur initiale positive de valeur grande par rapport à l'étendue de la plage, par exemple égale à un dixième de l'écart entre les valeurs extrêmes de la distance focale accessibles pour ce dispositif de capture d'images, ici 2 centimètres par exemple,

- le sens de modification de la distance focale, appelé SensFocus est initialisé dans le sens de l'augmentation de cette distance focale.

Inversement, on pourrait envisager que la distance focale du dispositif de capture d'image soit réglée à sa valeur maximale, et que le sens de modification de la distance focale soit initialement le sens de la diminution de cette distance.

Lors de l'étape de détection de la distance focale correspondant à une netteté maximum, on incrémente la distance focale du dispositif de capture d'image, et on répète les étapes j) et k) décrites précédemment, en utilisant, à l'étape k), une valeur seuil du maximum de la netteté de l'image prédéterminée.

Si la valeur mesurée sur l'image de la taille ou de la luminosité est supérieure à ladite valeur seuil du maximum de netteté, le programme est configuré pour relancer l'acquisition d'une nouvelle image (bloc 100).

Dans ce cas, le programme d'autofocus externe étant enclenché, les étapes de réglage grossier de la luminosité ne sont pas effectuées.

On détermine alors la position des éléments de repère (bloc 301) et on répète l'étape de détection du maximum de netteté des éléments de repère.

Si la valeur mesurée sur l'image de la taille ou de la luminosité est inférieure à ladite valeur seuil du maximum de netteté, la distance focale correspondant au maximum de netteté des éléments de repère est dépassée.

Le programme effectue alors une étape de changement de l'incrément et du sens de modification de la distance focale :

- l'incrément DeltaFocus est diminué, par exemple divisé par 2,

- le sens de modification de la distance focale SensFocus est inversé.

La distance focale est alors modifiée en diminuant la distance focale de la nouvelle valeur de l'incrément.

Après le démarrage du programme de réglage de la distance focale, on répète les étapes d'acquisition et de traitement d'image décrit précédemment (bloc 100, 101, 301, 302, 303, 304) jusqu'à ce que l'incrément DeltaFocus soit inférieur à une valeur prédéterminée.

Cette valeur prédéterminée est par exemple atteinte lorsque la différence entre deux pas successifs est inférieure à 10% de la valeur initiale de l'incrément.

En variante, à l'étape de détection de la netteté maximum, pour chaque image capturée successivement avec une valeur de distance focale augmentée de l'incrément DeltaFocus, on détermine la taille ou la luminosité de l'image d'un point particulier de l'élément de repère de profil et on en déduit le paramètre de netteté correspondant. Le programme compare pour chaque incrémentation de la distance focale, la valeur du paramètre de netteté avec la valeur du paramètre de netteté déterminée pour l'incrément précédent de la distance focale. La valeur du paramètre de netteté varie de manière continue de manière à tendre vers une valeur correspondant à un maximum de netteté. Lorsque le sens de variation de ce paramètre s'inverse, la distance focale correspondant à la netteté maximum est dépassée.

Les étapes suivantes sont inchangées : le programme effectue alors une étape de changement de l'incrément et du sens de modification de la distance focale, puis l'étape de détection du maximum de netteté est répétée avec ces valeurs initiales.

Le maximum est détecté avec suffisamment de précision lorsque la valeur de l'incrément devient inférieure à un seuil d'incrémentation. Lorsque cette valeur de l'incrément est atteinte, le programme conserve la valeur de distance focale correspondant à l'inversion de la variation du paramètre de netteté obtenu avec cet incrément comme valeur de distance focale pour les captures d'images ultérieures et le programme est stoppé.

De préférence, le réglage de netteté est effectué après le réglage grossier de la luminosité, et l'image de profil de la tête du porteur capturée à l'étape h), est capturée avec le paramètre de réglage des conditions optiques d'acquisition modifié à l'étape p3).

De préférence, à l'étape c1) suivante, l'image de profil de la tête du porteur est capturée avec la distance focale d'acquisition d'image modifiée à l'étape I).

*Réglage fin de la luminosité*

Ce réglage est par exemple réalisé ici après le réglage de la netteté des éléments de repère.

Après l'arrêt du programme de réglage de la distance focale d'acquisition, l'image suivante capturée présente un réglage grossier de luminosité satisfaisant et un réglage de netteté satisfaisant.

On procède alors à un réglage fin de la luminosité des images des éléments de repère (bloc 205, 206 de la figure 1).

Afin de permettre le réglage fin de la luminosité, l'accessoire 20 comporte au moins un repère de calibrage de luminosité comprenant au moins deux zones contrastées et visibles sur ladite image de profil du porteur.

Ici les éléments de calibrage de luminosité sont confondus avec les éléments de repère décrits précédemment. L'accessoire comporte donc quatre repères de calibrage de luminosité visibles sur une image sensiblement de face qui sont les éléments de repère de face 21D, 21G, 22H, 22B et deux repères de calibrage visibles sur une image sensiblement de profil droite ou gauche qui sont les élément de repère de profil 23, 24. Avantageusement, le procédé comporte les étapes suivantes, préalables à l'étape c1), pour assurer la capture d'une image sensiblement de profil de luminosité acceptable :

e) on capture une image sensiblement de profil de la tête du porteur (bloc 100 de la figure 1),
f) on détermine une valeur mesurée de la luminosité d'au moins une partie de l'image de l'élément de calibrage capturée à l'étape e) (bloc 205 de la figure 1), qui est ici l'image de l'un des éléments de repère,
g) on modifie un paramètre de réglage des conditions optiques d'acquisition d'image dudit dispositif de capture d'image en fonction de cette valeur mesurée, de manière à améliorer le contraste de l'image des zones contrastées de l'élément de calibrage (bloc 206 de la figure 1).

De préférence, à l'étape e), l'image sensiblement de profil de la tête du porteur est capturée avec le paramètre de réglage des conditions optiques d'acquisition modifié à l'étape I).

Comme précédemment, la luminosité peut être une valeur de luminance et/ou de chrominance selon les éléments de repère utilisés.

A l'étape f), on identifie la position des éléments de repère comme décrit plus haut, ou on utilise les positions identifiées dans une étape précédente. Une fois la position des éléments de repère déterminée, on détermine une valeur mesurée de la luminosité, luminance et/ou chrominance, d'au moins une partie de l'image de cet élément de repère de luminosité,

La partie de l'image de l'élément de calibrage de luminosité pour laquelle on détermine une valeur mesurée de la luminosité est appelée dans la suite la zone de mesure. Elle est par exemple située à cheval sur lesdites deux zones contrastées de l'élément de calibrage.

La valeur de luminosité mesurée est une valeur moyenne sur l'ensemble de la zone de mesure.

Ici cette mesure est effectuée dans une zone à cheval sur les quatre zones contrastées, de préférence centrée sur le centre de l'élément de calibrage.

A l'étape g), on réalise les sous-étapes suivantes :

g1) on détermine l'écart entre la valeur de luminosité déterminée à l'étape f) et une valeur cible de luminosité,
g2) on compare cet écart avec une valeur maximale prédéterminée de cet écart,
g3) en fonction de cette comparaison, on modifie le paramètre de réglage des conditions optiques d'acquisition du dispositif de capture d'image de manière à faire tendre ladite valeur mesurée vers ladite valeur cible de luminosité.

La valeur cible de luminosité est prédéterminée par une étape d'étalonnage préalable. Elle peut par exemple être déterminée à partir d'une première image capturée dans des conditions de luminosité optimale ou être déterminée à partir d'une valeur moyenne de valeur mesurée de la luminosité sur des images exploitables.

En pratique, si cet écart est supérieur à une valeur maximale prédéterminée de cet écart, la modification du paramètre de réglage des conditions optiques d'acquisition du dispositif de capture d'image est par exemple égale à la différence entre la valeur cible de luminosité et ladite valeur mesurée de la luminosité divisée par ladite valeur maximale prédéterminée de l'écart entre cette valeur cible de luminosité et cette valeur mesurée.

Si cet écart est inférieur à une valeur maximale prédéterminée de cet écart, la modification du paramètre de réglage est nulle et on peut alors passer directement à une vérification de la netteté, si celle-ci est réalisée après cette étape, ou à la détermination du paramètre géométrico-physionomique (bloc 400 de la figure 1).

Ladite valeur maximale prédéterminée de l'écart entre la valeur cible et la valeur mesurée de luminosité dépend par exemple du nombre de pas de réglage du paramètre de réglage des conditions optiques d'acquisition du dispositif de capture d'image. Plus ce paramètre de réglage comporte un grand nombre de pas de réglage, plus la valeur maximale prédéterminée de l'écart sera grande, afin de permettre néanmoins un réglage rapide de ce paramètre.

La modification du paramètre de réglage peut avantageusement dépendre de la valeur de luminosité mesurée, de la valeur cible et de la valeur courante du paramètre de réglage.

Le lien entre la luminosité et le paramètre de réglage n'est pas forcément linéaire sur les caméras. Par exemple, une petite modification du paramètre de réglage lorsque l'image est saturée ou sous-exposée peut rester sans effet visible.

Ainsi, il peut être avantageux de faire varier la valeur maximale prédéterminée de l'écart en fonction de la valeur courante du paramètre de réglage.

Le paramètre de réglage des conditions optiques d'acquisition du dispositif de capture d'image est un paramètre d'ouverture et/ou de gain et/ou de temps d'exposition.

En pratique, le dispositif de capture d'image est par exemple une caméra vidéo au format PAL entrelacé. La taille initiale de l'image, avant ré-échantillonnage est par exemple de 720x576 pixels.

On ajuste alors le paramètre de luminosité « bright » de la caméra

Le paramètre de luminosité n'est pas un paramètre physique de la caméra, mais une consigne. La caméra est par exemple utilisée en mode de réglage semi-automatique. En fonction du paramètre de luminosité demandé, la caméra ajuste alors automatiquement les trois paramètres matériels suivants :

- iris ou ouverture ou diaphragme,
- gain,
- temps de pause ou « shutter ».

A l'étape c1), l'image sensiblement de profil de la tête du porteur est de préférence capturée avec le paramètre de réglage des conditions optiques d'acquisition modifié à l'étape g).

En variante, si le réglage de netteté est effectué après le réglage de la luminosité, l'image de profil de la tête du porteur capturée à l'étape h), est capturée avec le paramètre de réglage des conditions optiques d'acquisition modifié à l'étape g).

Ainsi, l'étape de détermination de la valeur de référence de l'angle d'inclinaison peut être réalisée à partir d'une image sensiblement de profil ayant une luminosité et une netteté adaptée à l'obtention d'un résultat précis et l'étape de détermination du paramètre géométrico-physionomique (bloc 400) recherché est réalisée sur une image sensiblement de face dont la luminosité et la netteté sont également satisfaisantes.

Les étapes d'identification de la position des images des repères de l'accessoire, de réglage de la netteté et de la luminosité peuvent être mise en œuvre que ce soit pour les éléments de repère de profil que pour les éléments de repère de face. Ainsi, elles sont applicables à l'image sensiblement de profil pour permettre la détermination de la valeur de référence de l'angle d'inclinaison, comme à l'image de face pour permettre la détermination de la valeur mesurée de l'angle.

**Revendications**

1. Procédé de détermination, dans une posture naturelle dans laquelle le plan de Francfort PF est sensiblement horizontal, d'au moins un paramètre géométrico-physionomique associé au montage d'une lentille ophtalmique dans une monture (30) de lunettes destinée à être portée par un porteur, comportant les étapes suivantes :

    a) on capture au moins une image sensiblement de face de la tête (10) du porteur,
    b) on détermine une valeur mesurée (Am) d'un angle d'inclinaison (A) de la tête (10) du porteur, pendant la capture de l'image sensiblement de face, qui dépend de l'inclinaison de la tête du porteur autour d'un axe principal perpendiculaire à un plan sagittal de la tête du porteur,
    c) la tête du porteur étant placée dans ladite posture naturelle dans laquelle le plan de Francfort PF est sensiblement horizontal, on détermine une valeur de référence (Aref) dudit angle d'inclinaison (A) correspondant à ladite posture naturelle de la tête (10) du porteur, et
    d) on détermine ledit paramètre géométrico-physionomique recherché à partir de l'image sensiblement de face capturée et en fonction de la différence entre ladite valeur mesurée (Am) de l'angle d'inclinaison (A) déterminé à l'étape b) et ladite valeur de référence (Aref) dudit angle d'inclinaison (A) déterminée à l'étape c),

**caractérisé en ce que**, à l'étape c), on réalise les étapes suivantes :

c1) on capture, de manière non simultanée à la capture d'image de l'étape a), au moins une image sensiblement de profil de la tête du porteur placée dans ladite posture naturelle, la tête (10) du porteur comportant au moins un élément de repère de profil (23, 24) disposé de telle sorte qu'il soit identifiable sur ladite image sensiblement de profil de la tête du porteur,

c2) on identifie, sur ladite image sensiblement de profil, l'image de cet élément de repère de profil,

c3) on détermine une caractéristique géométrique de l'image de cet élément de repère de profil, et

c4) on détermine ladite valeur de référence (Aref) dudit angle d'inclinaison (A) correspondant à ladite posture naturelle de la tête (10) du porteur en fonction de ladite caractéristique géométrique déterminée à l'étape c3).

2. Procédé selon la revendication 1, selon lequel, à l'étape b), l'angle d'inclinaison (A) est l'angle formé entre un plan (PM) solidaire de la tête (10) du porteur et un plan de capture d'image (PCI) associé à la capture de ladite image sensiblement de face.

3. Procédé selon la revendication 2, selon lequel ledit plan (PM) solidaire de la tête (10) du porteur est un plan moyen (PM) des cercles de la monture (30) de lunettes disposée sur la tête de ce porteur.

4. Procédé selon l'une des revendications 1 à 3, selon lequel la tête du porteur comporte au moins un élément de repère frontal (21 D, 21G, 22H, 22B) disposé de telle sorte qu'il soit identifiable sur ladite image sensiblement de face du visage du porteur, et, à l'étape b) :

b1) on identifie sur cette image sensiblement de face l'image de l'élément de repère frontal,

b2) on détermine une caractéristique géométrique de l'image de cet élément de repère frontal,

b3) on détermine ladite valeur mesurée (Am) de l'angle d'inclinaison (A) associée à l'image sensiblement de face en fonction de la caractéristique géométrique déterminée à l'étape b2).

5. Procédé selon l'une des revendications 1 à 4, selon lequel on répète les étapes c1) à c4) et on détermine la valeur de référence (Aref) de l'angle d'inclinaison (A) comme la valeur moyenne des valeurs de référence déterminées lors d'une pluralité des étapes c4) réalisées.

6. Procédé selon l'une des revendications 1 à 5, selon lequel ledit élément de repère de profil (23, 24) et ledit élément de repère frontal (21 D, 21G, 22H, 22B) sont situés sur un accessoire (20) disposé sur la tête (10) du porteur.

7. Procédé selon l'une des revendications 1 à 6, selon lequel, la tête (10) du porteur comportant également au moins un repère de calibrage de luminosité (23, 24) comprenant au moins deux zones contrastées , visible sur ladite image de profil du porteur, le procédé comporte en outre les étapes suivantes, préalables à l'étape c) :

e) on capture une image sensiblement de profil de la tête du porteur,

f) on détermine une valeur mesurée de la luminosité d'au moins une partie de l'image de l'élément de calibrage capturée à l'étape e),

g) on modifie un paramètre de réglage des conditions optiques d'acquisition d'image dudit dispositif de capture d'image en fonction de cette valeur mesurée, de manière à améliorer le contraste de l'image des zones contrastées de l'élément de calibrage.

8. Procédé selon la revendication 7, selon lequel, à l'étape g),

g1) on détermine l'écart entre la valeur de luminosité déterminée à l'étape f) et une valeur cible de luminosité,

g2) on compare cet écart avec une valeur maximale prédéterminée de cet écart,

g3) en fonction de cette comparaison, on modifie le paramètre de réglage des conditions optiques d'acquisition du dispositif de capture d'image de manière à faire tendre ladite valeur mesurée vers ladite valeur cible de luminosité.

9. Procédé selon l'une des revendications 7 et 8, selon lequel, à l'étape c1), l'image sensiblement de profil de la tête (10) du porteur est capturée avec le paramètre de réglage des conditions optiques d'acquisition modifié à l'étape g).

10. Procédé selon l'une des revendications 1 à 9, comportant en outre les étapes suivantes, préalables à l'étape c) :

h) on capture une image sensiblement de profil de la tête (10) du porteur,

i) on détermine la position de l'image de l'élément de repère de profil (23, 24) sur cette image capturée à l'étape h),

j) on détermine un paramètre de netteté représentatif de la netteté de l'image de cet élément de repère de profil sur cette image,

k) on compare ce paramètre de netteté avec une valeur seuil de netteté prédéterminée,

l) on modifie la distance focale d'acquisition d'image dudit dispositif de capture d'image en fonction de la différence entre le paramètre de netteté déterminé à l'étape j) et ladite valeur seuil de netteté, de manière à améliorer la netteté de l'image de élément de repère de profil (23, 24).

11. Procédé selon la revendication 10, prise dans sa dépendance de la revendication 7, selon lequel, à l'étape h), l'image de profil de la tête du porteur est capturée avec le paramètre de réglage des conditions optiques d'acquisition modifié à l'étape g).

12. Procédé selon la revendication 10, prise dans sa dépendance de la revendication 7, selon lequel, à l'étape e), l'image de profil de la tête du porteur est capturée avec le paramètre de réglage des conditions optiques d'acquisition modifié à l'étape I).

13. Procédé selon l'une des revendications 10 à 12, selon lequel, à l'étape c1), l'image de profil de la tête du porteur est capturée avec la distance focale d'acquisition d'image modifiée à l'étape I).

14. Procédé selon l'une des revendications 10 à 13, selon lequel, à l'étape j), on détermine la taille ou la luminosité de l'image d'un point particulier de l'élément de repère de profil (23, 24) et on en déduit ledit paramètre de netteté.

15. Procédé selon l'une des revendications 7 à 9, selon lequel ledit élément de calibrage de luminosité de profil (23, 24) est confondu avec l'élément de repère de profil (23, 24).

## Patentansprüche

1. Verfahren zum Bestimmen in einer natürlichen Haltung, in der die Frankfurter Horizontale PF im Wesentlichen horizontal ist, wenigstens eines geometrisch-physiognomischen Parameters, der der Montage eines Brillenglases in einem Brillengestell (30), das von einem Träger getragen werden soll, zugeordnet ist, das die folgenden Schritte umfasst:

a) Aufnehmen wenigstens eines Bildes im Wesentlichen der Vorderseite des Kopfes (10) des Trägers,
b) Bestimmen eines Messwerts (Am) eines Neigungswinkels (A) des Kopfes (10) des Trägers während der Aufnahme des Bildes im Wesentlichen der Vorderseite, der von der Neigung des Kopfes des Trägers um eine Hauptachse senkrecht zu einer Sagittalebene des Kopfes des Trägers abhängt,
c) wenn der Kopf des Trägers in einer natürlichen Haltung angeordnet wird, in der die Frankfurter Horizontale PF im Wesentlichen horizontal ist, Bestimmen eines Referenzwerts (Aref) des Neigungswinkels (A), der der natürlichen Haltung des Kopfes (10) des Trägers entspricht, und
d) Bestimmen des gesuchten geometrisch-physiognomischen Parameters anhand des aufgenommenen Bildes im Wesentlichen der Vorderseite in Abhängigkeit von der Differenz zwischen dem Messwert (Am) des Neigungswinkels (A), der im Schritt b) bestimmt wird, und dem Referenzwert (Aref) des Neigungswinkels (A), der im Schritt c) bestimmt wird,

**dadurch gekennzeichnet, dass** im Schritt c) die folgenden Schritte ausgeführt werden:

c1) Aufnehmen nicht gleichzeitig mit der Aufnahme des Bildes des Schrittes a) wenigstens eines Bildes im Wesentlichen des Profils des Kopfes des Trägers, der in der natürlichen Haltung angeordnet ist, wobei der Kopf (10) des Trägers wenigstens ein Profilbezugselement (23, 24) aufweist, das in der Weise angeordnet ist, dass es in dem Bild im Wesentlichen des Profils des Kopfes des Trägers identifiziert werden kann,
c2) Identifizieren in dem Bild im Wesentlichen des Profils des Bildes dieses Profilbezugselements,
c3) Bestimmen einer geometrischen Charakteristik des Bildes dieses Profilbezugselements und
c4) Bestimmen des Referenzwertes (Aref) des Neigungswinkels (A), der der natürlichen Haltung des Kopfes (10) des Trägers entspricht, in Abhängigkeit von der geometrischen Charakteristik, die im Schritt c3) bestimmt wird.

2. Verfahren nach Anspruch 1, wobei im Schritt b) der Neigungswinkel (A) der Winkel ist, den eine mit dem Kopf (10) des Trägers fest verbundene Ebene (PM) mit einer Bildaufnahmeebene (PCI), die der Aufnahme des Bildes im Wesentlichen der Vorderseite zugeordnet ist, bildet.

3. Verfahren nach Anspruch 2, wobei die Ebene (PM), die mit dem Kopf (10) des Trägers fest verbunden ist, eine Mittelebene (PM) der Kreise des am Kopf dieses Trägers angeordneten Brillengestells (30) ist.

4. Verfahren nach einem der Ansprüche 1 bis 3, wobei der Kopf des Trägers wenigstens ein Frontbezugselement (21D, 21G, 22H, 22B) besitzt, das in der Weise angeordnet ist, dass es in dem Bild im Wesentlichen der Vorderseite des Gesichts des Trägers identifiziert werden kann, und wobei im Schritt b):

b1) in diesem Bild im Wesentlichen der Vorderseite das Bild des Frontbezugselements identifiziert wird,

b2) eine geometrische Charakteristik des Bildes dieses Frontbezugselements bestimmt wird,

b3) der Messwert (Am) des Neigungswinkels (A), der dem Bild im Wesentlichen der Vorderseite zugeordnet ist, in Abhängigkeit von der im Schritt b2) bestimmten geometrischen Charakteristik bestimmt wird.

5. Verfahren nach einem der Ansprüche 1 bis 4, wobei die Schritte c1) bis c4) wiederholt werden und der Referenzwert (Aref) des Neigungswinkels (A) als der Mittelwert der Referenzwerte, die in mehreren ausgeführten Schritten c4) bestimmt werden, bestimmt wird.

6. Verfahren nach einem der Ansprüche 1 bis 5, wobei das Profilbezugselement (23, 24) und das Frontbezugselement (21D, 21G, 22H, 22B) sich an einem Zubehör (20), das am Kopf (10) des Trägers angeordnet ist, befinden.

7. Verfahren nach einem der Ansprüche 1 bis 6, wobei der Kopf (10) des Trägers außerdem wenigstens einen Helligkeitskalibrierungsbezug (23, 24) aufweist, der wenigstens zwei Kontrastzonen enthält, die in dem Bild des Profils des Trägers sichtbar sind, wobei das Verfahren außerdem vor dem Schritt c) die folgenden Schritte umfasst:

e) Aufnehmen eines Bildes im Wesentlichen des Profils des Kopfes des Trägers,

f) Bestimmen eines Messwerts der Helligkeit wenigstens eines Teils des im Schritt e) aufgenommenen Bildes des Kalibrierungselements,

g) Modifizieren eines Parameters für die Einstellung optischer Bedingungen der Bilderfassung der Bildaufnahmevorrichtung in Abhängigkeit von diesem Messwert, derart, dass der Kontrast des Bildes der Kontrastzonen des Kalibrierungselements verbessert wird.

8. Verfahren nach Anspruch 7, wobei im Schritt g)

g1) der Abstand zwischen dem im Schritt f) bestimmten Helligkeitswert und dem Soll-Helligkeitswert bestimmt wird,

g2) dieser Abstand mit einem vorgegebenen Maximalwert für diesen Abstand verglichen wird,

g3) in Abhängigkeit von diesem Vergleich der Parameter für die Einstellung der optischen Bedingungen der Erfassung der Bildaufnahmevorrichtung in der Weise modifiziert wird, dass der Messwert zu dem Soll-Helligkeitswert strebt.

9. Verfahren nach einem der Ansprüche 7 und 8, wobei im Schritt c1) das Bild im Wesentlichen des Profils des Kopfes (10) des Trägers mit dem Parameter für die Einstellung der optischen Bedingungen der Aufnahme, der im Schritt g) modifiziert wird, aufgenommen wird.

10. Verfahren nach einem der Ansprüche 1 bis 9, das außerdem vor dem Schritt c) die folgenden Schritte umfasst:

h) Aufnehmen eines Bildes im Wesentlichen des Profils des Kopfes (10) des Trägers,

i) Bestimmen der Position des Bildes des Profilbezugselements (23, 24) in diesem im Schritt h) aufgenommenen Bild,

j) Bestimmen eines Schärfeparameters des Bildes dieses Profilbezugselements in diesem Bild,

k) Vergleichen dieses Schärfeparameters mit einem vorgegebenen Schärfeschwellenwert,

l) Modifizieren der Brennweite für die Erfassung des Bildes der Bildaufnahmevorrichtung in Abhängigkeit von der Differenz zwischen dem im Schritt j) bestimmten Schärfeparameter und dem Schärfeschwellenwert, derart, dass die Schärfe des Bildes des Profilbezugselements (23, 24) verbessert wird.

11. Verfahren nach Anspruch 10, wenn abhängig von Anspruch 7, wobei im Schritt h) das Bild des Profils des Kopfes des Trägers mit dem Parameter für die Einstellung der optischen Bedingungen für die Erfassung, der im Schritt g) modifiziert wird, aufgenommen wird.

12. Verfahren nach Anspruch 10, wenn abhängig von Anspruch 7, wobei im Schritt e) das Bild des Profils des Kopfes des Trägers mit dem Parameter für die Einstellung der optischen Bedingungen für die Erfassung, der im Schritt l) modifiziert wird, aufgenommen wird.

13. Verfahren nach einem der Ansprüche 10 bis 12, wobei im Schritt c1) das Bild des Profils des Kopfes des Trägers mit der Brennweite für die Erfassung des Bildes, die im Schritt l) modifiziert wird, aufgenommen wird.

14. Verfahren nach einem der Ansprüche 10 bis 13, wobei im Schritt j) die Größe oder die Helligkeit des Bildes eines bestimmten Punkts des Profilbezugselements (23, 24) bestimmt wird und daraus der Schärfeparameter abgeleitet wird.

15. Verfahren einem der Ansprüche 7 bis 9, wobei das Helligkeitskalibrierungselement (23, 24 des Profils mit dem Profilbezugselement (23, 24 zusammenfällt.

## Claims

1. A method for determining, in a natural posture in which the Frankfurt plane PF is substantially horizontal, at least one geometric/physiognomic parameter associated with the mounting of an ophthalmic lens in a spectacle frame (30) intended to be worn by a wearer, comprising the following steps:

   a) capturing at least one substantially frontal image of the wearer's head (10),
   b) determining a measured value (Am) of an angle of inclination (A) of the wearer's head (10), during the capture of the substantially frontal image, which depends on the inclination of the wearer's head about a main axis perpendicular to a sagittal plane of the wearer's head,
   c) the wearer's head being placed in said natural posture in which the Frankfurt plane PF is substantially horizontal, determining a reference value (Aref) of said angle of inclination (A) corresponding to said natural posture of the wearer's head (10), and
   d) determining said looked-for geometric/physiognomic parameter on the basis of the substantially frontal image captured and as a function of the difference between said measured value (Am) of the angle of inclination (A) determined in step b) and said reference value (Aref) of said angle of inclination (A) determined in step c),

   **characterized in that**, in step c), the following steps are carried out:

   c1) capturing, in a manner that is not simultaneous to the image capture of step a), at least one substantially profile image of the wearer's head placed in said natural posture, the wearer's head (10) comprising at least one profile indexing element (23, 24) positioned in such a way that it is identifiable on said substantially profile image of the wearer's head,
   c2) identifying, on said substantially profile image, the image of this profile indexing element,
   c3) determining a geometric characteristic of the image of this profile indexing element, and
   c4) determining said reference value (Aref) of said angle of inclination (A) corresponding to said natural posture of the wearer's head as a function of said geometric characteristic determined in step c3).

2. The method as claimed in claim 1, whereby, in step b), the angle of inclination (A) is the angle formed between a plane (PM) integral with the wearer's head (10) and an image capture plane (PCI) associated with the capture of said substantially frontal image.

3. The method as claimed in claim 2, whereby said plane (PM) integral with the wearer's head (10) is a mean plane (PM) of the circles of the spectacle frame (30) positioned on this wearer's head.

4. The method as claimed in one of claims 1 to 3, whereby the wearer's head comprises at least one frontal indexing element (21D, 21G, 22H, 22B) positioned in such a way that it is identifiable on said substantially frontal image of the face of the wearer, and, in step b):

   b1) the image of the frontal indexing element is identified on this substantially frontal image,
   b2) a geometric characteristic of the image of this frontal indexing element is determined,
   b3) said measured value (Am) of the angle of inclination (A) associated with the substantially frontal image is determined as a function of the geometric characteristic determined in step b2).

5. The method as claimed in one of claims 1 to 4, whereby steps c1) to c4) are repeated and the reference value (Aref) of the angle of inclination (A) is determined as the average value of the reference values determined in a plurality of steps c4) carried out.

6. The method as claimed in one of claims 1 to 5, whereby said profile indexing element (23, 24) and said frontal indexing element (21D, 21G, 22H, 22B) are situated on an accessory (20) positioned on the wearer's head (10).

7. The method as claimed in one of claims 1 to 6, whereby, the wearer's head (10) also comprising at least one brightness calibration indexer (23, 24) comprising at least two contrasted areas, visible in said profile image of the wearer, the method also comprises the following steps, prior to step c):

   e) a substantially profile image of the wearer's head is captured,
   f) a measured value of the brightness of at least a portion of the image of the calibration element captured in step e) is determined,
   g) a setting parameter of the optical image acquisition conditions of said image capture device is modified as a function of this measured value, so as to improve the contrast of the image of the contrasted areas of the calibration element.

8. The method as claimed in claim 7, whereby, in step g),
   g1) the deviation between the brightness value determined in step f) and a target brightness value is determined,
   g2) this deviation is compared with a predetermined maximum value of this deviation,

g3) based on this comparison, the setting parameter of the optical acquisition conditions of the image capture device is modified so as to make said measured value tend toward said target brightness value.

(23,24).

9. The method as claimed in one of claims 7 and 8, whereby, in step c1), the substantially profile image of the wearer's head (10) is captured with the setting parameter of the optical acquisition conditions modified in step g).

10. The method as claimed in one of claims 1 to 9, also comprising the following steps, prior to step c): h) a substantially profile image of the wearer's head (10) is captured,

i) the position of the image of the profile indexing element (23, 24) on this image captured in step h) is determined,

j) a sharpness parameter representative of the sharpness of the image of this profile indexing element on this image is determined,

k) this sharpness parameter is compared with a predetermined sharpness threshold value,

1) the image acquisition focal distance of said image capture device is modified as a function of the difference between the sharpness parameter determined in step j) and said sharpness threshold value, so as to improve the sharpness of the image of the profile indexing element (23, 24).

11. The method as claimed in claim 10 taken in its dependency with claim 7, whereby, in step h), the profile image of the wearer's head is captured with the setting parameter of the optical acquisition conditions modified in step g).

12. The method as claimed in claim 10 taken in its dependency with claim 7, whereby, in step e), the profile image of the wearer's head is captured with the setting parameter of the optical acquisition conditions modified in step 1).

13. The method as claimed in one of claims 10 to 12, whereby, in step c1), the profile image of the wearer's head is captured with the image acquisition focal distance modified in step 1).

14. The method as claimed in one of claims 10 to 13, whereby, in step j), the size or the brightness of the image of a particular point of the profile indexing element (23, 24) is determined, and said sharpness parameter is deduced therefrom.

15. The method as claimed in one of claims 7 to 9, whereby said profile brightness calibration element (23, 24) is merged with the profile indexing element

# Fig.1

Acquisition vidéo ⌐ 100

Autofocus externe en cours ? ⌐ 101

Non | Oui

**Algorithme asservissement luninosité**

Analyse luminosité zones latérales ⌐ 201

Modification réglage luminosité — Oui

Non | 202

Détection mires

203

Test netteté mires — Pas OK

Ok | 204

Analyse luminosité mires — 205

Modification réglage luminosité — Oui

206

Non

**Algorithme autofocus externe**

Démarrage autofocus externe

300 | 301

Détection mires

Détection maximum netteté — Non

302

Chargement incrément/sens focus — Non

303 | Oui

Modification Focus

305 | 304

Fin Autofocus externe

Détermination paramètre géométrico-physionomique ⌐ 400

Fig.2

Fig.3

Fig.4

Fig.5

Fig.6A

Fig.6B

L

ZC

ZS

21G

H

ZC

ZS

PFr

At1

At

PS

A

PF

AH

PM

At2

PCl

BH

Fig.7

## RÉFÉRENCES CITÉES DANS LA DESCRIPTION

**Documents brevets cités dans la description**

- WO 2010119190 A1
- JP 2007289683 A
- WO 2010145736 A1
- US 12596351 B